# EUROPEAN PATENT SPECIFICATION

(11) **EP 1 997 795 B1**
(45) Date of publication and mention of the grant of the patent: **09.03.2016**
(21) Application number: 08105289.6
(22) Date of filing: 05.04.2001
(51) Int. Cl.: C07C 43/313, C07C 43/315, C08F 16/38, C07C 41/50

(54) **Fluorovinylethers and polymers obtainable therefrom**
Fluorvinylether und daraus hergestellte Polymere
Ethers fluorovinyliques et des polymères ainsi obtenus

(30) Priority: 21.04.2000 IT MI20000902
(43) Date of publication of application: 03.12.2008
(62) Divisional of application: 01108596.6
(73) Proprietor: Solvay Specialty Polymers Italy S.p.A., 20121 Milano (IT)
(72) Inventor: Navarrini, Walter, 20010 Boffalora Ticino (MI) (IT)
(74) Representative: Benvenuti, Federica

(56) References cited:
- EP-A- 0 683 181
- EP-A- 0 976 706
- WO-A-99/48939

## Description

The present invention relates to fluorovinylethers, the process for preparing them and the polymers obtainable therefrom.

It is well known that perfluoroalkylvinylethers are used as monomers for the olefin copolymerization, specifically tetrafluoroethylene, vinylidene fluoride, chlorotrifluoroethylene (CTFE), hexafluoropropene. The introduction of small amounts of perfluoroalkylvinylethers in plastomeric polymers implies a higher polymer processability and better hot mechanical properties. The introduction of high amounts of perfluorovinylethers in crosslinkable fluoropolymers implies elastomeric properties at low temperature of fluorinated rubbers.

The need was felt, in the fluorinated polymeric material field, to produce both plastomers having good properties at high temperatures, and elastomers having improved properties at low temperatures by using only one fluorovinylether.

Such properties at low temperatures can generally be expressed by the glass transition temperature Tg.

Furthermore the need was felt to have available amorphous or crystalline (co)polymers having a low content of COF end groups. A lower content of COF end groups leads to obtain polymers having a higher thermal stability. A lower Tg allows to have polymers which can be used at lower temperatures and therefore to have available elastomers with a wider use range. To obtain the combination of the above mentioned properties, fluorovinylethers must have a high unitary capability to modify the backbone properties, as well as high reactivity to be used as comonomers both in plastomeric and in elastomeric fluoropolymers. It was desirable to have available vinylethers obtainable by simple processes having a limited number of steps. Preferably it would be desirable to have available a continuous process for preparing said vinylethers.

To solve the above technical problem, fluorovinylethers having different structural properties, have been proposed in the prior art. However from the prior art, hereinafter described, various unsolved problems exist in the perfluorovinylether synthesis and in the preparation of the corresponding polymers having the combination of the above mentioned properties.

USP 3,132,123 describes the preparation of perfluoroalkylvinylethers, of the corresponding homopolymers and copolymers with TFE. Homopolymers are obtained under extreme experimental conditions, by using polymerization pressures from 4,000 to 18,000 atm. The perfluoromethylvinylether (PMVE) homopolymer is an elastomer: the Tg is not reported.

The general formula of the described vinylethers is the following:

CF₂=CFOR⁰_{F}

wherein R⁰_{F} is a perfluoroalkyl radical preferably from 1 to 5 carbon atoms. A process for preparing these vinylethers is described in USP 3,291,843 wherein the starting acylfluoride is salified and pyrolized with carbonates also in the presence of solvents. By this process undesired hydrogenated by-products are obtained.

USP 3,450,684 relates to vinylethers having the formula:

CF₂=CFO(CF₂CFX⁰O)_{n'}CF₂CF₂X⁰

wherein X⁰ = F, Cl, CF₃, H and n' can range from 1 to 20.
Also homopolymers obtained by UV polymerization are reported. The exemplified copolymers are not characterized by their properties at low temperatures.

USP 3,635,926 relates to the emulsion copolymerization of perfluorovinylethers with TFE, showing that the presence of -COF acylfluoride end groups makes the polymers unstable. The same phenomenon was already reported in USP 3,085,083 in the perfluorovinylether polymerization systems in solvent.

USP 3,817,960 relates to the preparation and polymerization of perfluorovinylethers having the formula

CF₃O(CF₂O)_{n"}CF₂CF₂OCF=CF₂

wherein n" can range from 1 to 5. The compound synthesis is complex, it requires three steps. The preparation of the starting compound CF₃O(CF₂O)_{n"}CF₂C(O)F is carried out by oxidation at low temperature in the presence of U.V. radiations; besides the condensation with HFPO (hexafluoropropenoxide) and the subsequent alkaline pyrolisis is necessary. No data on the above indicated properties are reported. With regard to this see USP 5,910,552.

USP 3,896,179 relates to the separation of "primary" isomers of perfluorovinylethers, for example of CF₃CF₂CF₂OCF=CF₂ from the corresponding less stable "secondary" isomers CF₃-(CF₃)CFOCF=CF₂. The latter are undesired products as regards both the polymer preparation and the poor properties of the obtained polymers.

USP 4,340,750 relates to the preparation of perfluorovinylethers having the formula

CF₂=CFOCF₂R⁰_{f}X¹

wherein R⁰_{f} is a C₁-C₂₀ perfluoroalkyl optionally containing oxygen, X¹= H, Cl, Br, F, COOR⁰, CONR⁰R' wherein R⁰ is a C₁-C₁₀ alkyl group and R' represents H or a C₁-C₁₀ alkyl group. In the preparation of these compounds an acylfluoride together with iodine and tetrafluoroethylene is used, avoiding the final step of the acylfluoride pyrolisis which comes from the perfluoropropene epoxide, by a deiodofluorination reaction, which takes place with low yields.

USP 4,487,903 relates to the preparation of fluoroelastomeric copolymers using perfluorovinylethers having the formula:

CF₂=CF(OCF₂CFY⁰)ₙ⁰OX²

wherein n° ranges from 1 to 4; Y⁰= F, Cl, CF₃, H; X² can be C₁-C₃ perfluoroalkyl group, C₁-C₃ ω-hydroperfluoroalkyl group, C₁-C₃ ω-chloroperfluoroalkyl group. The polymer has a content of fluorovinylether units ranging from 15 to 50% by moles. These vinylethers give copolymers which at low temperatures have better properties than those of the above mentioned perfluorovinylethers PVE (perfluoropropylvinylether) and MVE type. In the patent it is disclosed that in order to have good properties at low temperature, the presence of at least two ether bonds in the side chain adjacent to the double bond is required. Furthermore from the patent it results that for n⁰ values higher than 4 it is difficult to purify the monomers and the effect on the decrease of the polymer Tg is lower. Besides the reactivity of the described vinylethers is very low and it is difficult to obtain polymers having a high molecular weight able to give good elastomeric properties. A TFE/perfluorovinylether copolymer (n⁰= 2) 73/27 % by moles with Tg of -32°C is exemplified. However the polymer is obtained with very long reaction times (96 hours of polymerization).

EP 130,052 describes the perfluorovinylpolyether (PVPE) polymerization which leads to amorphous perfluoropolymers with a Tg ranging from -15 to -100°C. The described polymers have Tg values reaching up to -76°C; the further Tg decrease is obtained by using perfluoropolyethers as plasticizers. In the patent copolymers and terpolymers of TFE and MVE with vinylethers (PVPE) having the formula

CF₂=CFO(CF₂CF(CF₃)O)_{n"'}R⁰_{f'}

are described, wherein n ranges from 3 to 30 and R⁰_{f'} is a perfluoroalkyl group. Due to purification difficulties, the used vinylethers are vinylether mixtures with different n values. According to said patent the most evident effect on the Tg decrease is shown when n is equal to or higher than 3, preferably higher than 4. According to the polymerization examples described in said patent the final mass of the polymer, besides the hot and under vacuum treatment, must then be washed with Freon^{®} TF in order to remove all the unreacted monomer (PVPE). From the Examples it results that the reactivity of all the described monomers (PVPE) is poor.

USP 4,515,989 relates to the preparation of new intermediates for the fluorovinylether synthesis. According to the patent the vinylether synthesis is improved by using an intermediate able to more easily decarboxylate. For its preparation fluoroepoxides of formula: 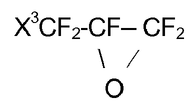 wherein X³ = Cl, Br are used.

USP 4,619,983 describes the copolymerization of VDF with vinylethers having the formula:

CF₂=CFOX⁴

wherein X⁴ is a C₃-C₉ oxyperfluoroalkyl radical containing from 1 to 3 oxygen atoms. The obtained polymers are not perfluorinated polymers and show a poor stability to alcohols.

USP 4,766,190 relates to the polymerization of perfluorovinylpolyethers (PVPE) similar to those of USP 4,487,903 with TFE and low perfluoropropene percentages, in order to increase the mechanical properties of the obtained polymers.

EP 338,755 relates to the preparation of perfluorinated copolymers by using direct fluorination of partially fluorinated copolymers. More reactive partially fluorinated monomers are used, the obtained polymers are fluorinated with elemental fluorine. The fluorination step requires a supplementary process unit, besides in this step elemental fluorine is used, which is a highly oxidizing gas, with the consequent precautions connected to its use. Besides in the patent it is stated that in order not to compromise the fluorination reaction and the properties of the obtained polymer, using the invention process the percentage of the comonomer in the polymer cannot exceed 50% by moles.

USP 5,268,405 reports the preparation of perfluorinated rubbers having a low Tg, by the use of high viscosity perfluoropolyethers as plasticizers of perfluorinated rubbers (TFE/MVE copolymers). However during the use perfluoropolyether bleeds take place. This is true especially for the PFPE having a low molecular weight (low viscosity): in said patent, therefore, the high viscosity PFPE use is suggested, and therefore the low viscosity PFPEs must previously be removed. USP 5,350,497 relates to the preparation of perfluoroalkylvinylethers by fluorination with elemental fluorine of hydrofluorochloroethers and subsequent dechlorination.

USP 5,401,818 relates to the prepartion of perfluorovinylethers of formula:

R¹_{f}(OCF₂CF₂CF₂)_{m'}-OCF=CF₂

(wherein R¹_{f} is a C₁-C₃ perfluoroalkyl radical and m' is an integer ranging from 1 to 4) and of the corresponding copolymers having improved properties at low temperature. The preparation of said perfluorovinylethers is carried out by 7 steps, some of them have very low yields, and comprise also a fluorination with elemental F₂. The reactivity of said perfluorovinylethers is anyhow low.

As it is shown from the above prior art, the perfluorovinylether synthesis generally involves a multistep process with low yields (USP 3,132,123, USP 3,450,684), with additional purifications to remove undesired isomers (USP 3,896,179) and the need to control the undesired hydrogenated by-products (USP 3,291,843). Alternatively, in the synthesis substances acting as intermediates, which are suitably prepared, and which allow to eliminate said drawbacks (USP 4,340,750, USP 4,515,989), are used.

Furthermore in some cases the vinylether preparation requires the fluorination with elemental fluorine of partially fluorinated intermediates (USP 5,350,497); or, to avoid synthesis and low reactivity problems of the perfluorovinylethers, fluorination of partially fluorinated polymers (EP 338,755) is suggested.

Other problems shown in the prior art relate to the low reactivity of the perfluorovinylethers, which makes it necessary the recovery of the unreacted monomers from the reaction products (UK 1,514,700), and the stability problems for the polymers having -C(O)F end groups (USP 3,635,926). These last can be furtherly transformed by suitable reactants in order to increase the stability of the fluorinated polymer (EP 178,935).

Perfluorooxyalkylvinylethers are used to confer to the fluorinated rubbers good properties at low temperatures, and specifically to lower the copolymer glass transition temperature.

By increasing the perfluorooxyalkyl units forming the side perfluorooxyalkyl substituent, the Tg of the corresponding obtainable amorphous copolymers decreases, but at the same time the vinylether reactivity drastically decreases, making more evident the problems previously shown for the recovery of the unreacted monomer from the polymerization products or from the polymer itself (USP 4,487,903 - EP 130,052). In some cases, where the monomer cannot be completely removed by simple stripping under vacuum, more washings must then be carried out with fluorinated solvents for completely eliminating the unreacted vinylethers from the polymeric mass.

The perfluoromethylvinylether (MVE) is used as comonomer in plastomeric fluoropolymers and, at higher concentrations, also in elastomeric fluoropolymers. In particular in EP 633,257 and EP 633,274 MVE is polymerized with TFE in the presence of small amounts of PVE or dioxoles to obtain polymers with improved flex life.

The amorphous copolymers of TFE with perfluoromethylvinylether have a Tg around 0°C or slightly lower (Maskornik, M. et al. "ECD-006 Fluoroelastomer - A high performance engineering material". Soc. Plast Eng. Tech. Pao. (1974), 20, 675-7).

The Tg extrapolated value of the MVE homopolymer is of about -5°C (J. Macromol. Sci.-Phys., B1(4), 815-830, Dec. 1967).

In USP 5,296,617 and 5,235,074 there is described the hypofluorite CF₂(OF)₂ reactivity towards unsaturated products, which contemporaneously leads to the formation of the dioxolane derivative and to the fluorination compound of the olefin itself. In EP 683,181 there is described the CF₂(OF)₂ reactivity towards olefins which leads to the formation of linear reaction compounds between one hypofluorite molecule and two molecules of the same olefin, for the preparation of symmetric dienes.

WO 99/48939 discloses perfluoroelastomer compositions comprising peroxide curable perfluorolastomers that are said to be characterized by improved processability and lower reactivity to bases (page 4, lines 4 to 6).

The perfluoroelastomer has copolymerized units of perfluoroolefin, a perfluorovinyl ether and a cure site-component having at least one bromine or iodine-containing moiety (page 4, lines 6 to 10). The perfluoroelastomer provides compositions that are said to be processable at ambient temperature without forming a crumbly mass (page 10, lines 31 - 32), endowed with lower viscosity and also with excellent thermal stability and chemical resistance (page 11, lines 10-11 and 14-15).

However, it is noted that this document is not concerned with the obtainment of amorphous or crystalline (co)polymers having a lower content of -COF end groups, which is responsible for higher thermal stability.

WO 99/48939 teaches that different perfluorinated vinyl ethers can be used for the manufacture of the perfluoroelastomer, for example those falling within general formulae (I) to (V) reported on page 4, line 15 to page 15, line 10.

Among specific examples of perfluorinated vinyl ethers, one of formula CF₂=CFOCF₂OCF₂CF₂CF₃ is mentioned (page 5, line 8); nevertheless, this ether does not fall within any one of formulae (I) to (V) and this document does not give any teaching or hint on how to prepare such ether.

EP 0976706 relates to hydro-fluoroalkylvinylethers and processes for the manufacture thereof. In particular, this document discloses hydro-2,2-di-fluoroalkylvinylethers having the formula:

CF₂=CH-O-R_{A} (I)

wherein R_{A} is an alkyl radical containing fluorine, optionally containing halogens such as Cl, Br, I (par. [0013]). This document teaches that the hydro-fluoroalkylvinylethers can be copolymerized with other monomers to provide polymers and copolymers. (par. [0017]), but it does not provide any teaching concerning the relevance of the hydro-fluoroalkylvinylethers on the properties of the resulting polymers and copolymers.

The Applicant has surprisingly and unexpectedly found that it is possible to solve the above technical problem as described hereinafter, by using special fluorovinylethers, which are furthermore easily synthetizable and obtainable by a continuous process.

An object of the present invention are fluorovinylethers having the following formula:

CFX=CXOCF₂OR

wherein R is a C₂-C₆ linear, branched (per)fluorooxyalkyl group containing from one to three oxygen atoms; when R is a fluorooxyalkyl group as above defined it can contain from 1 to 2 atoms, equal to or different from each other, selected from the following: H, Cl, Br, I; X = F.

Preferably fluorovinylethers have formula:

CFX=CXOCF₂OCF₂CF₂Y

wherein Y = OCF₃; X as above defined. Surprisingly, the vinylethers according to the invention show the advantages reported hereinafter with respect to the known vinylethers.

The obtainable advantages can be attributed to the -OCF₂O- unit directly bound to the ethylene unsaturation.

The Tg lowering obtained with the vinylethers of the invention is connected to the presence of the (-OCF₂O-) unit directly bound to the unsaturation. The Tg lowering is surprisingly so evident to be defined a primary effect.

In fact, if a vinylether with two oxygen atoms is used:

CF₂=CF-O-CF₂-O-CF₂CF₃ (MOVE 1)

the Tg lowering is clearly higher with respect to the PVE

CF₂=CF-O-CF₂CF₂CF₃ (PVE)

and to the vinylether having the same formula, but with the second oxygen atom in a different position and without showing the characteristic unit (-OCF₂O-)

CF₂=CF-O-CF₂CF₂-O-CF₃ (β-PDE)

It is surprising to notice that with respect to MVE

CF₂=CF-O-CF₃

the β-PDE vinylether does not give any advantage as regards Tg.

On the contrary the primary effect of the (-OCF₂O-) unit results very clear with the vinylethers of the invention (MOVE).

Besides it has surprisingly been found that the (-OCF₂O-) unit bound to the ethylene unsaturation of the vinylethers of the invention increases the vinylether reactivity drastically reducing the rearrangements to COF which cause instability.

The advantages of the invention can be summarized as follows:
- The reactivity of the new monomers allows to prepare copolymers having a high MW (molecular weight) with a very low content of carboxylic groups or derivatives thereof such as -C(O)F, -COO-. The carboxylic group content in the copolymer with TFE resulted about 10 times lower than that of a copolymer prepared under the same conditions but using perfluoropropylvinylether (PVE) instead of fluorovinylethers (see the Examples). As said, the presence of a lower content of carboxylic groups, or of the corresponding derivatives (amides, esters, etc.) allows to obtain more stable polymers.
- The reactivity of the fluorovinylethers of the invention is surprisingly high (see the homopolymerization Examples).
- The fluorovinylethers of the invention can be used as comonomers both in plastomeric (per)fluoropolymers (containing crystalline domains) and in elastomeric (per)fluoropolymers. To obtain plastomeric polymers the amount of the vinylether of the invention must be such to lead to the formation of crystalline domains, generally < 10% by moles. To obtain amorphous polymers the amount of the vinylether of the invention must be such to lead to the disappearance of the crystalline domains. The skilled man in the art can easily find the amount of the vinylether of the invention which is required for obtaining said results.
   Generally the amount of the vinylether for obtaining amorphous polymers is higher than 10% by moles, preferably in the range from about 15 to 20% by moles, or higher. In the case of copolymers having a high content of vinylether monomer, the properties at low temperature (Tg) of the polymers of the invention result clearly better with respect both to copolymers having the same MVE content (see the Examples) and also, surprisingly, with respect to copolymers where the perfluorovinylether, the oxygen atoms being equal, does not show the -OCF₂O- group directly bound to the unsaturation, as in the case of the CF₂=CFOCF₂CF₂OCF₃ (β-PDE) (see the Examples).
- The use of the fluorovinylethers of the invention in the polymerization reactions with fluoroolefins allows to substantially and contemporaneously reduce two important disadvantages of the prior art: the recovery of the unreacted vinylether and the polymer instability due to the presence of carboxylic end groups.
- A further advantage of the fluorovinylethers of the invention, as hereinafter illustrated, consists in that their preparation is carried out in a continuous plant by a limited number of steps. Furthermore the used raw materials are cheap. The following ones can for example be mentioned:
   CF₂(OF)₂, CF₂=CF₂, CF₂=CFOCF₃, CHCl=CFCl, CFCl=CFCl, CF₂=CFCl, CF₂=CFH, CF₂=CH₂, CHCl=CHCl and other olefins. The use of these reactants is specified in the synthesis process of the vinylethers of the invention.

Polymers, homopolymers, copolymers are obtainable by polymerizing the fluorovinylethers of general formula (I)-(IV) with at least another monomer.

With copolymer, a polymer containing the vinylether of the invention and one or more comonomers, is meant.

With comonomers, fluorinated compounds having at least one polymerizable double bond C=C, optionally containing hydrogen and/or chlorine and/or bromine and/or iodine and/or oxygen, are preferably meant.

Optional comonomers which can be copolymerized are non fluorinated C₂-C₈ olefins, such as ethylene, propylene, isobutylene.

Among the usable comonomers the following can be mentioned:
- C₂-C₈ perfluoroolefins, such as tetrafluoroethylene (TFE), hexafluoropropene (HFP), hexafluoroisobutene;
- C₂-C₈ hydrogenated fluoroolefins, such as vinyl fluoride (VF), vinylidene fluoride (VDF), trifluoroethylene, CH₂=CH-R²_{f} perfluoroalkylethylenes, wherein R²_{f} is a C₁-C₆ perfluoroalkyl;
- C₂-C₈ chloro- and/or bromo- and/or iodo-fluoroolefins, such as chlorotrifluoroethylene (CTFE) and bromotrifluoroethylene;
- CF₂=CFOR²_{f} (per)fluoroalkylvinylethers (PAVE), wherin R²_{f} is a C₁-C₆ (per)fluoroalkyl, for example trifluoromethyl, bromodifluoromethyl or heptafluoropropyl;
- CF₂=CFOX^{a} (per)fluoro-oxyalkylvinylethers, wherein X^{a} is: a C₁-C₁₂ alkyl, or a C₁-C₁₂ oxyalkyl, or a C₁-C₁₂ (per)fluorooxyalkyl having one or more ether groups, for example perfluoro-2-propoxy-propyl;
- sulphonic monomers having the structure CF₂=CFOX^{b}SO₂F, wherein X^{b} = CF₂CF₂, CF₂CF₂CF₂, CF₂CF(CF₂X^{c}) wherein X^{c} = F, Cl, Br.

The process for preparing fluorinated polymers from the fluorovinylethers according to the present invention can be carried out by polymerization in organic solvent as described in USP 4,864,006 and 5,182,342, herein incorporated by reference. The organic solvent is selected from the group comprising chlorofluorocarbons, perfluoropolyethers, hydrofluorocarbons and hydrofluoroethers.

The process for preparing the polymers can be carried out also by polymerization in aqueous emulsion according to well known methods in the prior art, in the presence of a radical initiator. This can be selected for example from: inorganic peroxides (for example alkaline metal or ammonium persulphates, perphosphates, perborates or percarbonates), optionally in combination with ferrous, cuprous or silver salts, or of other easily oxidizable metals; organic peroxides (for example, disuccinylperoxide, terbutyl-hydroperoxide, diterbutylperoxide); azocompounds (see USP 2,515,628 and USP 2,520,338, herein incorporated by reference). It is also possible to use organic or inorganic redox systems, such as ammonium persulphate/sodium sulphite, hydrogen peroxide/aminoiminomethansulphinic acid.

In the reaction medium also surfactants of various type are usually present, among which the fluorinated surfactants of formula:

R³_{f}-X⁻ M⁺

are particularly preferred, wherein R³_{f} is a C₅-C₁₆ (per)fluoroalkyl chain or a (per)fluoropolyoxyalkyl chain, X⁻ is -COO⁻ or -SO₃⁻, M⁺ is selected from: H⁺, NH₄⁺, an alkaline metal ion. Among the most commonly used, ammonium perfluorooctanoate, (per)fluoropolyoxyalkylenes ended with one or more carboxylic groups, etc. can be mentioned.

During the polymerization, known iodinated chain transfer agents can be added to the reaction medium. It is also possible to use as chain transfer agents alkaline or earth-alkaline metal iodides and/or bromides, according to USP 5,173,553, herein incorporated by reference.

Other chain transfer agents are mentioned in USP 4,766,190, herein incorporated by reference.

The crosslinking process of the amorphous polymers can be carried out according to well known methods in the prior art. When, for example, one of the comonomers is vinylidene fluoride or vinyl fluoride, curing can be carried out with polyamines or aromatic polyols in the presence of suitable catalysts (accelerants) as described in USP 3,876,654, USP 4,259,463; when the monomer is perfluorinated, one generally uses the addition in amounts lower than or equal to 3% of a comonomer having a reactive site formed, for example, by Br, I, CN, OC₆F₅, COOR^{a} wherein R^{a} is an alkyl from 1 to 5 carbon atoms, or by double bonds as described in USP 5,268,405; when the polymer contains Br or I, it is cured in the presence of a peroxide or of a polyunsaturated compound as described in USP 4,948,852, USP 4,948,853, USP 4,983,697, -EP 683,149.

Another object of the present invention consists in the synthesis process of the new (per)fluorovinylethers having formula:

CFX=CXOCF₂OR (I)

wherein R is a C₂-C₆ linear, branched (per)fluoro oxyalkyl group containing from one to three oxygen atoms; when R is a fluorooxyalkyl group as above defined it can contain from 1 to 2 atoms, equal to or different from each other, selected from the following: H, Cl, Br, I; X = F,
and

CFX=CXOCF₂OCF₂CF₂Y (II)

wherein Y = F, OCF₃; X as above defined,
comprising the reaction of the hypofluorite with a fluorinated olefin of formula R₁R₂C=CR₃R₄ to give the intermediate hypofluorite F-CR₁R₂-CR₃R₄-OCF₂OF, the subsequent reaction of said compound with a second fluorinated olefin of formula R₅R₆C=CR₇R₈ to give the intermediate F-CR₁R₂-CR₃R₄-OCF₂O-CR₅R₆-CR₇R₈-F, which by dehalogenation or dehydrohalogenation leads to the new perfluorovinylethers.
The general scheme of the synthesis is the following:
a)

   CF₂(OF)₂ + R₁ R₂C=CR₃R₄ → F-CR₁R₂-CR₃R₄-OCF₂OF (VI)
b)

   F-CR₁R₂-CR₃R₄-OCF₂OF + R₅R₆C²=C¹R₇R₈ → F-CR₁R₂-CR₃R₄-OCF₂O-C²R₅R₆-C¹R₇R₈-F (VII)
c)

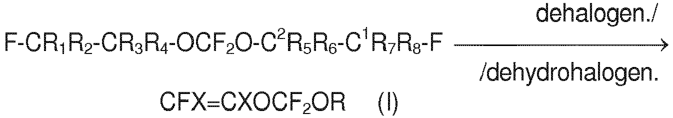

In this synthesis scheme:
- with reference to the formula of the compound (VII):
- R₁ and R₄, equal or different, are F; R₂, R₃, equal or different are H, Cl under the following conditions: (1) when the final reaction is a dehalogenation R₂, R₃ = Cl, (2) when the final reaction is a dehydrohalogenation one of the two substituents R₂, R₃ is H and the other is Cl;
- R₅, R₆, R₇, R₈ are:
   - F, or one of them is a C₁-C₄ linear or branched perfluorooxyalkyl group containing from one to three oxygen atoms;
   - when one of the R₅ to R₈ radicals is a C₂-C₄ linear or branched fluorooxyalkyl group containing from one to three oxygen atoms, one or two of the other R₅ to R₈ are F and one or two of the remainders, equal to or different from each other, are selected from H, Cl, Br, Iodine; when the substituents selected from H, Cl, Br, Iodine are two, they are both linked to the same carbon atom;
- The fluorinated olefine used in the reaction a) is replaceable with that of the subsequent reaction b); in this case the meanings defined for the substituents of the R₁-R₄ group, and respectively of the R₅-R₈ group, are interchangeable each other, with the proviso that the position of each radical of each of the two groups R₁-R₄ and R₅-R₈ with respect to -OCF₂O- on the chain of the intermediate compound (VII), is the same which is occupied if the synthesis takes place according to the above reported scheme and the two olefins each react in the considered steps.

In the first reaction a) of the above illustrated scheme a hypofluorite gas flow CF₂(OF)₂, suitably diluted with an inert fluid, comes into contact, in a suitable reactor with outlet, on the bottom of the same (first reactor), with a flow of the R₁R₂C=CR₃R₄ olefin, optionally diluted in an inert fluid, to allow the chemical reaction a) with formation of the intermediate hypofluorite (VI). To favour the reaction stoichiometry, the reactants must be introduced into the reactor in an approximately unitary molar ratio, or with an excess of CF₂(OF)₂. The residence time of the mixture in the reactor can range from few hundredths of second up to about 120 seconds depending on the olefin reactivity, the reaction temperature and the presence of optional reaction solvents.

The reaction temperature can range from -40° to -150°C, preferably from-80° to -130°C.

The compound (VI) usually is not separated from the reaction product and it is transferred in a continuous way to the subsequent reaction described in step b).

The mixture of the products coming out from the first reactor can be heated at room temperature before being fed into the second reactor.

In the second reaction b) the second olefin R₅R₆C=CR₇R₈, pure or in solution, reacts with the product obtained in the first reaction with formation of compound (VII).

The olefin can be fed in a continuous way, so as to maintain its concentration constant in the reactor. The temperature of the reaction b) can range from -20° to -130°C, preferably from -50° to -100°C. The olefin concentration is higher than or equal to 0.01 M, preferably the concentration is higher than 3M, more preferably also the pure compound can be used.

The solvents used in steps a) and b) are perfluorinated or chlorohydrofluorinated solvents or hydrofluorocarbons. Examples of said solvents are: CF₂Cl₂, CFCl₃, CF₃CFH₂, CF₃CF₂CF₃, CF₃CCl₂H, CF₃CF₂Cl.

In the reaction c) the compound (VII), dependently on the olefins used in steps a) and b), after distillation from the reaction product, is subjected to dechlorination or to dehydrochlorination to obtain the vinylethers of formula (I).

This last step can be carried out by using reactions widely described in the prior art. The suitable selection of the substituents R₁ to R₈ in the two olefins used in the synthesis allows to obtain the vinylethers of the present invention.

The following Examples are reported with the purpose to illustrate the invention and they do not limit the scope of the same.

In the Examples the thermogravimetric analysis TGA is carried out by using a 10°C/min rate.

### EXAMPLE 1 (Reference example)

### Synthesis of CF₃CF₂OCF₂OCFClCF₂Cl perfluoro-1-2,dichloro-3,5-dioxaheptane

The used reactor is of cylindrical type, with a total volume of 300 ml and is equipped with magnetic dragging mechanical stirrer, turbine with recycle of the reacting gas placed at 20 cm from the reactor top, internal thermocouple, two internal copper pipes for the reactant feeding which end at about 1 mm from the turbine, and product outlet from the bottom. In the reactor, inside of which the temperature is maintained at -114°C, 1.1 l/h (litres/hour) of CF₂(OF)₂ and 3.3 l/h of He are introduced through one of the two inlet pipes; A flow of 1.1 l/h of CF₂=CF₂ and 0.7 l/h of He is maintained through the second inlet pipe. Feeding is continued for 6.6 hours.

The residence time of the transport gas in the reaction zone comprised between the outlet of the two feeding pipes in the reactor and the inlet of the discharge pipe is of about 4 sec.

On the reactor bottom the reaction products are brought to room temperature and the gaseous mixture flow, monitored by gaschromatography, is fed in a continuous way, under mechanical stirring, into a second reactor having a 250 ml volume maintained at the temperature of -70°C, equipped with mechanical stirrer, thermocouple, dipping inlet for the reacting mixture, outlet with head of inert gas. The reactor contains 72.6 g of dichlorodifluoroethylene CFCI=CFCI.

At the end of the addition of reacting gases into the second reactor, the reaction raw material is distilled by a plate column at atmospheric pressure, collecting 41.5 g of the desired product (boiling point 91 °C).

The yield of perfluoro-1,2 dichloro-3,5-dioxaheptane, calculated with respect to CF₂(OF)₂, is of 36%.

### Characterization of prfluoro 1,2, dichloro-3,5-dioxaheptane

Boiling point at atmospheric pressure: 91 °C.

¹⁹F-NMR spectrum in p.p.m. (with respect to CFCl₃= O):
- 51.3/-53.0 (2F, O-CF₂-O); -70.6/-72.6 (2F, C-CF₂Cl); -78.0/-78.4 (1 F, O-CFCI-C); -87.8 (3F, CF₃-C); -90.2/-91.8 (2F, C-CF₂-O).

Mass spectrum (E.I. electronic impact), main peaks and respective intensities:
69 (48.6%); 119 (84.3%); 151 (76.8%); 153 (69.8%); 185 (100%).

IR spectrum (cm⁻¹) intensity: (w) = weak, (m) = medium, (s) = strong, (vs) = very strong:
1407.3 (w); 1235.8 (vs); 1177.7 (vs); 1097.5 (vs); 1032.2 (s); 929.3 (w); 847.9 (m).

### EXAMPLE 2

### Synthesis of CF₃OCF₂CF₂OCF₂OCFClCF₂Cl perfluoro-1,2-dichloro-3,5,8-trioxanonane (isomer A) and CF₃OCF(CF₃)OCF₂OCFClCF₂Cl perfluoro-1,2-dichloro-3,5,7-trioxa-6-methyloctane (isomer B)

In a reactor identical to that used in Example 1, maintained at the same temperature of -114°C, 1.55 l/h of CF₂(OF)₂ and 4.5 l/h of He are introduced through one of the two inlet pipes; through the second inlet pipe 1.4 l/h of CF₂=CF-OCF₃ and 0.7 l/h of He for 4.5 hours.

The residence time of the transport gas in the reaction zone comprised between the reactor outlet and the end of the two feeding pipes is of about 3 sec.

On the reactor bottom the reaction products are brought to room temperature and the gaseous mixture flow, monitored by gaschromatography, is fed in a continuous way, under mechanical stirring, into a second reactor identical to the one used for the same step in Example 1. Inside, where a temperature of -70°C is maintained, there are 51 g of dichlorofluoroethylene CFCI=CFCI.

At the end of the addition of the reacting gases into the second reactor, the reaction raw material is distilled by a plate column at the reduced pressure of 250 mmHg. 50 g of a mixture formed by two isomers, respectively, isomer A) perfluoro-1,2-dichloro-3,5,8-trioxanonane and isomer B) perfluoro-1,2-dichloro-3,5,7-trioxa-6-methyloctane are collected. The mixture composition is determined by gaschromatography and is the following: isomer A 79%, isomer B 21%. The molar yield of A+B with respect to the used CF₂(OF)₂ is 38%. The molar yield of A+B with respect to the used perfluoromethylvinylether is 42%. The isomers have been separated by preparative gaschromatography.

### Characterization of products A and B

Mixture boiling point (A 79%, B 21%) at the reduced pressure of 250 mmHg: 82°C.

¹⁹F-NMR spectrum in p.p.m. (with respect to CFCl₃=0) of the isomer A: -50.6/-52.4 (2F, O-CF₂-O); -70.0/-71.8 (2F, C-CF₂Cl); -77.7 (1 F, O-CFCI-C); -55.3/-55.6 (3F, CF₃-OC); -90.7/-91.1 (2F, C-OCF₂-C); -90.2/-90.6 (2F, C-OC-CF₂OCOC).

¹⁹F-NMR spectrum in p.p.m. (with respect to CFCl₃=0) of isomer B: -50.0/-52.1 (2F, O-CF₂-O); -70.0/-71.8 (2F, C-CF₂Cl); -77.9 (1 F, O-CFCI-C); -54.6/-54.9 (3F, CF₃OC): -85.7/-86.1 (3F, OC(CF₃)O); -100.3/-101.0 (1F, OCF(C)O).

Mass spectrum (electronic impact) main peaks and respective intensities: Product A: 69 (50); 119 (100); 151 (50); 185 (42); 251 (38);
Product B: 69 (96); 97 (50); 135 (42); 151 (92); 185 (100).

IR spectrum (cm⁻¹), intensity of the mixture A 79%, B 21%: (w) = weak, (m) = medium, (s) = strong, (vs) = very strong:
1388 (w); 1288 (vs); 1233 (vs); 1151 (vs); 1104 (vs); 1032 (s); 846 (m); 685 (w).

### EXAMPLE 3

### Synthesis of CF₃OCF₂CF₂OCF₂OCHClCHFCl perfluoro-1,2-dichloro-1,2-dihydro-3,5,8-trioxanonane (isomer C) and CF₃OCF(CF₃)OCF₂OCHClCHFCl perfluoro-1,2-dichloro-1,2-dihydro-3,5,7-trioxa-6-methyloctane (isomer D)

In a reactor identical to that used in Example 1, maintained at the temperature of -112°C, 1.55 l/h of CF₂(OF)₂ and 4.5 l/h of He are introduced through one of the two inlet pipes; through the second inlet pipe 1.4 l/h of CF₂=CF-OCF₃ and 0.7 l/h of He for 5 hours.

The residence time of the transport gas in the reaction zone comprised between the reactor outlet and the end of the two feeding pipes is of about 3 sec.

On the reactor bottom the reaction products are brought to room temperature and the gaseous mixture flow, monitored by gaschromatography, is fed in a continuous way, under mechanical stirring, into a second reactor identical to the one used for the same step in Example 1. Inside, the temperature is of -70°C and there are 50 g of 1,2-dichloroethylene CClH=CClH and 50 g of CFCl₃.

At the end of the addition of the reacting gases into the second reactor, after distillation of the solvent at room pressure, the reaction raw material is distilled by a plate column at the reduced pressure of 100 mmHg. 43.5 g of the mixture of the desired products (isomer C 78%, isomer D 22%, determined by gaschromaography) are collected. The molar yield of C+D with respect to the used CF₂(OF)₂ is 33%. The isomers have been separated by preparative gaschromatography.

### Characterization of products C and D

Mixture boiling point (C 78%, D 22%) at the reduced pressure of 100 mmHg: 71°C.

¹⁹F-NMR spectrum in p.p.m. (with respect to CFCl₃ = 0) of the isomer C perfluoro-1,2-dichloro-1,2-dihydro-3,5,8-trioxanonane: -56.0/-57.2 (2F, O-CF₂-O); -143.2/-146.0 (1F, C-CHFCl); -55. 8(3F, CF₃-OC);-91.0/-91.4 (2F, C-OCF₂-C); -90.3/-90.5 (2F, C-OC-CF₂OCOC).

¹⁹F-NMR spectrum in p.p.m. (with respect to CFCl₃=0) of the isomer D perfluoro-1,2-dichloro-1,2-dihydro-3,5,7-trioxa-6-methyloctane: -56.0/-57.2 (2F, O-CF₂-O); -143.2/-146.0 (1F, C-CHFCl); -54.9/-55.1 (3F, CF₃-OC); -86.2/-86.3 (3F, OC(CF₃)O); -100.5/-101.0 (1 F, OCF(C)O).

¹H spectrum in p.p.m. (with respect to TMS) of the isomers C and D: 6.28/6.05 (1H -CHFCl); 6.02/5.95 (1H -CHCl-).

Mass spectrum (electronic impact), main peaks and respective intensities %: 69 (84); 119 (100); 185 (51.1); 251 (84); 281 (15.8); 283 (4.8); 347 (5.7); 349 (1.7).

IR spectrum (cm⁻¹) intensity : (w) = weak, (m) = medium, (s) = strong, (vs) = very strong:
3001.0 (w); 2920.9 (w); 2850.9 (w); 1286.3 (vs); 1233.7 (vs); 1125.5 (vs); 1081.8 (s); 1047.9 (s); 815.9 (m); 766.3 (m).

### EXAMPLE 4 (Reference example)

### Dehaloqenation of perfluoro 1,2-dichloro-3,5-dioxaheptane

In a 25 ml three-necked flask, equipped with mechanical stirrer, thermometer, dropping funnel, distillation column equipped with water refrigerant and collecting trap maintained at -78°C and connected to a mechanical vacuum pump, 150 ml of DMF, 15 g of Zn in powder, 0.5 g of K₂CO₃ and 100 mg of I₂ are introduced. The internal temperature is brought to 80°C and 50 g of perfluoro-1,2-dichloro-3,5-dioxaheptane are added drop by drop. When the addition is over the mixture is allowed to react for about 30 minutes. At the end the internal pressure gradually brought from starting 760 mmHg to 300 mmHg. After about 20 minutes the collecting trap containing 34.2 g of perfluoro-3,5-dioxa-1-heptene (MOVE 1) is disconnected.

The dehalogenation yield is 85%.

### Characterization of perfluoro-3,5-dioxa-1-heptene (MOVE 1)

Boiling point at atmospheric pressure: 41.9°C.

¹⁹F-NMR spectrum in p.p.m. with respect to CFCl₃=0:
- 56.8 (2F, O-CF₂-O); -87.2 (3F, CF₃-C); -90.6 (2F, C-CF₂-O); -114 (1 F, O-C=C-F); -121.8 (1 F, O-C=CF); -137 (1 F, O-C-F=C).

Mass spectrum (electronic impact), main peaks and respective intensities: 69 (66.5%); 119 (100%); 147 (83.4%); 185 (89.4%); 216 (67.3%); 282 (8.2%).

IR spectrum (cm⁻¹) intensity: (w) = weak, (m) = medium, (s) = strong, (vs) = very strong:
1839.5 (m); 1407.6 (w); 1307.4 (vs); 1245.8 (vs); 1117.4 (vs); 907.2 (m); 846.0 (m).

### EXAMPLE 5

### Dehalogenation of the isomer mixture A+B obtained in Example 2 (perfluoro- 1,2-dichloro-3,5,8-trioxanonane CF₃OCF₂CF₂CF₂OCFClCF₂Cl + perfluoro-1,2-dichloro-3,5,7-trioxa-6-methyloctane CF₃OCF(CF₃)OCF₂OCFClCF₂Cl)

In a 250 ml flask equipped as described in the previous Example 4, 110 ml of DMF, 10 g of Zn in powder and 0.3 ml of Br₂ are introduced. The internal temperature is brought to 75°C and 30.3 g of the binary mixture A+B separated in the previous Example 2 are added drop by drop. When the addition is over the mixture is allowed to react for about 3 hours. At the end the internal pressure is gradually lowered from 760 mmHg to 200 mmHg at -79°C. After about 30 minutes the collecting trap is disconnected. The corresponding content, which is washed with water, is recovered. At the end 24.0 g of a mixture formed for 79% (gaschromatographic determination) by perfluoro-3,5,8-trioxa-1-nonene (MOVE 2) CF₃OCF₂CF₂OCF₂OCF=CF₂ (isomer A') and for 21 % by perfluoro-3,5,7-trioxa-6,methyl-1 octene (MOVE 2a) CF₃OCF(CF₃)OCF₂O-CF=CF₂ (isomer B') are obtained, which are then separated by preparative gaschromatography.

### Characterization of products A' and B'

Boiling range of the isomer mixture at atmospheric pressure: 72.5°-74.5°C.

¹⁹F-NMR spectrum in p.p.m. (with respect to CFCl₃=0) of the isomer A': -55.9 (3F, CF₃-O); -56.9 (2F, O-CF₂-O); -90.8 (2F, C-CF₂-O); -91.2 (2F, O-CF₂-C); -114 (1 F, O-C=C-F); -121.8 (1 F, -O-C=CF); -137 (1 F, O-CF=C).

¹⁹F-NMR spectrum in p.p.m. (with respect to CFCl₃=0) of the isomer B': -55.9 (3F, CF₃-O); -56.2 (2F, O-CF₂-O); -86.4 (3F, CF₃-C); -100.9 (1F, CF); -114 (1 F, O-C=C-F); -122 (1 F, O-C=CF); -137 (1 F, O-CF=C).

Mass spectrum (electronic impact), main peaks and respective intensities of the isomer A':
69 (74); 81 (18); 119 (100); 147 (59); 185 (26); 251 (21).

Mass spectrum (electronic impact), main peaks and respective intensities of the isomer B':
69 (80); 81 (37); 97 (47); 119 (36); 147 (100); 185 (19).

IR spectrum (cm⁻¹), intensity: (w) = weak, (m) = medium, (s) = strong, (vs) = very strong:
1839 (m); 1343 (s); 1248 (vs); 1145 (vs); 918 (m); 889 (m).

### EXAMPLE 6

### Dehalogenation of the isomers C+D mixture obtained in Example 3 (CF₃OCF₂CF₂OCF₂OCHClCHFCl perfluoro-1,2-dichloro-1,2-dihydro-3,5,8-trioxanonane (isomer C) + CF₃OCF(CF₃)OCF₂OCHClCHFCl perfluoro-1,2-dichloro-1,2-dihydro-3,5,7-trioxa-6-methyloctane (isomer D))

In a 500 ml three-necked flask, equipped with mechanical stirrer, thermometer, dropping funnel, distillation column having a water refrigerant and a collecting trap maintained at the temperature of -78°C, 250 ml of DMF, 30 g of zinc in powder and 300 mg of I₂ are introduced.

The temperature is brought to 100°C and 56.9 g of the isomer mixture obtained in Example 3 are added drop by drop. When the addition is over the reactor internal temperature is brought to 120°C and stirring is maintained for 24 hours. At the end the reaction product, which contains traces of solvent and which is collected in the trap maintained at -78°C, is distilled. After washing with water 35 g of a mixture of perfluoro-1,2-dihydro-3-5-8-trioxa-1-nonene (isomer C', 79% by moles) and of perfluoro-1,2-dihydro-3-5-7-trioxa-5-methyl-1-octene (isomer D', 21% by moles) are recovered. The isomers are separated by preparative gaschromatography.

The dehaloagenation reaction yield is 76%.

### Characterization of products C' and D'

Boiling range of the mixture of isomers C' 79%, D' 21% at atmospheric pressure: 90.0°-92.0°C.

¹⁹F-NMR spectrum in p.p.m. (with respect to CFCl₃=0) of the isomer C' perfluoro-1,2-dihydro-3,5,8-trioxa-1-nonene:
-55.7 (3F, CF₃-O); -57.3 (2F, O-CF₂-O); -90.9 (2F, C-CF₂-O) ; -91.2 (2F, O-CF₂-C); -149.3/-150.0 (1 F, O-C=C-F).
¹⁹F-NMR spectrum in p.p.m. (with respect to CFCl₃=0) of the isomer D' perfluoro-1,2-dihydro-3,5,7-trioxa-6-methyl-1-octene:
   -55.0 (3F, CF₃-O); -56.9 (2F, O-CF₂-O); -86.2 (3F, CF₃-C); -101.0 (1F, CF); -149.3/-150,0 (1 F, O-C=C-F)

Mass spectrum (electronic impact), main peaks and respective intensities %:
69 (82); 119 (100); 185 (29); 246 (25); 251 (20); 312 (43).

IR spectrum (cm⁻¹) intensity of the isomer mixture (C' 79%, D' 21%): (w) = weak, (m) = medium, (s) = strong, (vs) = very strong:
3140 (w); 1722 (w); 1695 (w); 1402 (m); 1281 (vs); 1237 (vs); 1147 (vs); 1106 (vs); 1030 (m).

### EXAMPLE 7 (Reference example)

### Homopolymerization of perfluoro-3,5-dioxa-1-heptene (MOVE 1)

In a glass reactor for polymerizations, having a 20 ml volume, equipped with magnetic stirring and with an inlet for the reactant feeding and discharge, 60 µl of perfluoropropionylperoxide at 3% by weight in CFCl₂CF₂Cl and 3 g of MOVE 1 are in sequence introduced. The so charged reactor is brought to the temperature of -196°C, evacuated, brought to room temperature, the all twice. At the end of the degassing operations the reactor is thermostated at the temperature of 30°C and the mixture is allowed to react under these conditions for two days under magnetic stirring.

The reaction raw material which is finally recovered appears as a slightly viscous, transparent, colourless and homogeneous solution.

After distillation of the unreacted monomer, and subsequent stripping under vacuum at 150°C for 3 hours, 180 mg of the polymer are separated.

The IR analysis of the obtained polymer shows that, in the spectrum, absorption bands in the zone of the fluorinated double bonds are absent.

The ¹⁹F-NMR analysis carried out on the polymer dissolved in C₆F₆ is in accordance with the homopolymer structure having a molecular weight of 50,000. The analysis does not show the presence of unreacted monomer.

The DSC graph does not show any melting endothermic curve, wherefore the polymer is amorphous. The polymer Tg, determined by DSC, is -35.4°C. The thermogravimetric analysis (TGA) shows a weight loss of 2% at 332°C and of 10% at 383°C.

### EXAMPLE 8

### Copolymer between perfluoro-3,5,8-trioxa-1-nonene CF₃OCF₂CF₂OCF₂OCF=CF₂ (MOVE 2) and perfluoro-3,5,7-trioxa-6, methyl-1-octene CF₃OCF(CF₃)OCF₂O-CF=CF₂ (MOVE 2a)

In a reactor having the same characteristics as that described in Example 7, 150 µl of perfluoropropionylperoxide at 3% by weight in CFCl₂CF₂Cl and 3.2 g of a mixture prepared according to the process of Example 5 and containing 83% MOVE 2 and 17% MOVE 2a, are introduced. The reactor is then evacuated, cooled, and the subsequent reaction carried out as described in the previous Example 7.

The reaction raw material appears as a slightly viscous, transparent, colourless and homogeneous solution. The monomers which have not reacted are distilled and a stripping under vacuum at 150°C for 3 hours is in sequence carried out. Finally 350 mg of the polymer are separated.

The IR analysis shows that, in the polymer spectrum, absorption bands in the zone of the fluorinated double bonds are absent.

The ¹⁹F-NMR analysis is in accordance with the copolymer structure having an average molecular weight of 35,000 and a MOVE 2/MOVE 2a content equal to the percentages of the reacting mixture; unreacted monomers are not evident. The DSC graph does not show any melting endothermic curve, wherefore the polymer is amorphous. The polymer Tg, determined by DSC, is -52.6°C. The thermogravimetric analysis (TGA) shows a weight loss of 2% at 280°C and of 10% at 327°C.

### EXAMPLE 9 (Reference example)

### Crystalline copolymer between MOVE 1 and TFE

A 5 l steel AISI 316 autoclave with stirrer working at 650 rpm has been used. After the vacuum has been effected, 3 l of demineralized water, 15.70 g of MOVE 1 and the microemulsion prepared according to the procedure described in USP 4,864,006 are in sequence introduced, so as to have a concentration of 2 g of surfactant/l of water.

The autoclave is heated up to 75°C and then 0.32 bar of ethane are introduced. A gaseous mixture in a molar ratio of 54.55 TFE/MOVE 1 is pumped by a compressor until having inside the autoclave a pressure of 21 absolute bar.

The composition of the gaseous mixture present in the autoclave top is analyzed by gaschromatography.

Before the reaction starting the gaseous phase results to be formed by the following molar percentages of the reactants: 93.1% TFE, 5.5% MOVE 1 and 1.4% Ethane. The reaction is triggered by feeding in a continuous way by a metering pump a potassium persulphate 0.0031 molar solution at a flow rate of 88 ml/h.

The pressure is maintained constant by feeding the monomer mixture. The polymer synthesis is stopped after 742 g of mixture have been fed in total.

The reactor is cooled at room temperature, the emulsion is discharged and the coagulation is induced by HNO₃ (65%) addition.

The polymer is separated, washed with water and dried at 220°C.

The IR analysis shows the presence of very small absorption bands in the carboxyl zone, whose intensity results to be half of the one obtained from a TFE/PVE copolymer film having the same thickness, prepared according to the comparative Example 3. MFI according to ASTMD 1238-52T was 4.4. The polymer therefore results thermally more stable (see the comparative Example hereunder).

### EXAMPLE 10

### Amorphous copolymer between MOVE 1 and TFE (Reference example)

In an AISI-316 polymerization reactor having a 40 ml volume, equipped with magnetic stirring, pressure transducer and an inlet for the reactant feeding and discharge, 250 µl of perfluoropropionylperoxide at 3% by weight in CFCl₂CF₂Cl, 9.8 mmoles of MOVE 1 and 18 mmoles of tetrafluoroethylene are introduced.

The reactor is cooled to the temperature of -196°C, evacuated, then brought to room temperature and cooled again, the all twice.

At the end of the degassing operations the reactor is thermostated at the temperature of 30°C and the reaction mixture maintained under magnetic stirring. The internal pressure decreases from 6.4 atm to 4.7 atm in about 8 hours (reaction time).

After distillation of the unreacted monomers, and polymer stripping under vacuum for 3 hours at 150°C, 1,100 mg of polymer are recovered, which appears as a transparent and colourless rubber.

By ¹⁹F-NMR analysis of the polymer dissolved under heating in C₆F₆ it is determined that the MOVE 1 molar percentage in the polymer is 24%.

The IR analysis does not show, in the polymer spectrum, absorption bands in the zone of the fluorinated double bonds, and shows the presence of very small absorption bands in the zone of the carboxyl signals. The intensity of these signals, compared with the similar ones obtained from a film having the same thickness obtained with the polymer of the comparative Example 1, is equal to about 1/10 of these latter.

The DSC graph does not show any melting endothermic curve, wherefore the polymer is amorphous. The Tg determined by DSC is -21.4.

The TGA shows a weight loss of 2% at 450°C and of 10% at 477°C. The polymer therefore results thermally more stable (see the comparative Example hereunder) with respect to the comparative Example (see afterwards).

The polymer intrinsic viscosity measured at 30°C in Fluorinert^{®} FC-75 is 35.5 ml/g.

### EXAMPLE 11 (Reference example)

### Amorphous copolymer between MOVE 1 and TFE

In an AISI-316 polymerization reactor identical to that described in the previous Example 10, 250 µl of perfluoropropionylperoxide at 3% by weight in CFCl₂CF₂Cl, 9.75 mmoles of MOVE 1 and 9 mmoles of tetrafluoroethylene are in sequence introduced.

The procedure already described in the previous Example 10 is followed until the thermostating step at the temperature of 30°C under magnetic stirring. During the reaction the internal pressure decreases from 3.4 atm to 2.9 atm in about 8 hours.

At the end the unreacted monomers are distilled and the polymer is stripped under vacuum at 150°C for 3 hours.

480 mg of the polymer are separated.

By ¹⁹F-NMR analysis of the polymer dissolved under heating in C₆F₆ it is determined that the MOVE 1 molar percentage in the polymer is 39%.

The IR analysis shows that, in the polymer spectrum, absorption bands in the zone of the fluorinated double bonds are absent.

The DSC graph does not show any melting endothermic curve, wherefore the polymer is amorphous. The Tg determined by DSC is -29.8°C.

The TGA shows a weight loss of 10% at 435°C.

### EXAMPLE 12 (Reference example)

### Amorphous copolymer between MOVE 1 and CF₂=CH₂

In a polymerization reactor identical to that described in Example 10, 250 µl of perfluoropropionylperoxide at 3% by weight in CFCl₂CF₂Cl, 10 mmoles of MOVE 1 and 18 mmoles of VDF are in sequence introduced.

The procedure already described in the previous Example 10 is followed until the thermostating step at the temperature of 30°C under magnetic stirring. The internal pressure decreases from 6.8 atm to 5.0 atm during the reaction (about 8 hours).

After distillation of the unreacted monomers, and subsequent polymer stripping under vacuum at 150°C for 3 hours, 1,600 mg of the polymer are separated, appearing as a transparent and colourless rubber.

By the ¹⁹F-NMR analysis carried out on the polymer dissolved in C₆F₆ it is determined that the MOVE 1 molar percentage in the polymer is 40%.

The DSC graph does not show any melting endothermic curve, wherefore the polymer is amorphous. The Tg determined by DSC is -47°C.

The TGA shows a weight loss of 2% at 428°C and of 10% at 455°C.

### EXAMPLE 13

### Amorphous terpolymer MOVE 2/MOVE 2a/TFE

In a polymerization reactor identical to that described in Example 10, 100 µl of perfluoropropionylperoxide at 6% by weight in CFCl₂CF₂Cl, 10 mmoles of a MOVE 2 (83%) and MOVE 2a (17%) mixture sinthetized according to the process of Example 5, and 18 mmoles of tetrafluoroethylene (TFE) are in sequence introduced.

The procedure already described in the previous Example 10 is then followed until thermostating at the temperature of 30°C under magnetic stirring. The internal pressure decreases from 6.1 atm to 3.9 atm during the reaction (about 8 hours).

After distillation of the unreacted monomers and polymer stripping under vacuum at 150°C for 3 hours, 1,131 mg of the polymer are separated.

By the ¹⁹F-NMR analysis carried out on the polymer dissolved in C₆F₆ it results that the total molar percentage of the MOVE 2 + MOVE 2a perfluorovinylethers in the polymer is 22%; the MOVE 2/MOVE 2a ratio by moles in the polymer is 83/17 and it is equal to that of the starting fed mixture.

The presence of unreacted monomers is not evident.

The IR analysis does not show, in the polymer spectrum, absorption bands in the zone of the fluorinated double bonds, and it shows the presence of very small absorption bands in the zone of the carboxyl signals. The intensity of these signals, compared with the similar ones obtained from a film having the same thickness obtained with the polymer of the comparative Example 1, is equal to about 1 /10 of the latter.

The DSC graph does not show any melting endothermic curve, wherefore the polymer is amorphous. The Tg determined by DSC is -37.5°C.

The TGA shows a weight loss of 10% at 473°C.

The polymer intrinsic viscosity measured at 30°C in Fluorinert^{®} FC-75 is 40.0 ml/g.

### EXAMPLE 14

### Amorphous terpolymer MOVE 2/MOVE 2a/TFE

In a polymerization reactor identical to that described in Example 10, 100 µl of perfluoropropionylperoxide at 6% by weight in CFCl₂CF₂Cl, 9.7 mmoles of the MOVE 2 (83%) and MOVE 2a (17%) mixture sinthetized according to the process of Example 5, and 10 mmoles of tetrafluoroethylene (TFE) are in sequence introduced.

The procedure already described in the previous Example 10 is then followed until the thermostating step at the temperature of 30°C under magnetic stirring. The internal pressure decreases from 3.6 atm to 2.7 atm during the reaction course (about 8 hours).

After distillation of the unreacted monomers and polymer stripping under vacuum at 150°C for 3 hours 652 mg of polymer are separated.

By the ¹⁹F-NMR analysis carried out on the polymer dissolved in C₆F₆ it results that the total molar percentage of the MOVE 2 + MOVE 2a perfluorovinylethers in the polymer is 37%; the MOVE 2/MOVE 2a molar ratio in the polymer is 83/17 and it is equal to that of the starting fed mixture.

The presence of unreacted monomers is not evident.

The IR analysis does not show, in the polymer spectrum, absorption bands in the zone of the fluorinated double bonds.

The DSC graph does not show any melting endothermic curve, wherefore the polymer is amorphous. The Tg determined by DSC is -44.5°C.

The TGA shows a weight loss of 10% at 451 °C.

The polymer intrinsic viscosity measured at 30°C in Fluorinert^{®} FC-75 is 16.7 ml/g.

### EXAMPLE 15

### Amorphous copolymer between perfluoro-1,2-dihydro-3,5,8-trioxa-1-nonene (H-MOVE 2) and perfluoro-1,2-dihydro-3,5,7-trioxa-6-methyl-1-octene (H-MOVE 2a) with molar ratio 88/12

In a reactor identical to that described in Example 7, 200 µl of perfluoropropionylperoxide at 3% by weight in CFCl₂-CF₂Cl and 3.1 g of a H-MOVE 2/H-MOVE 2a 88/12 mixture are introduced.

The procedure described in Example 7 is followed.

The recovered reaction raw material appears as a slightly viscous, transparent, colourless and homogeneous solution.

After distillation of the unreacted monomer and subsequent stripping under vacuum at 150°C for 3 hours, 120 mg of polymer are separated.

The IR analysis of the obtained polymer shows that, in the polymer spectrum, absorption bands in the zone of the fluorinated double bonds are absent.

The ¹⁹F-NMR analysis is in accordance with the copolymer structure having a content of monomers H-MOVE 2 and H-MOVE 2a equal to the H-MOVE 2 and H-MOVE 2a percentages in the reacting mixture. The analysis does not show the presence of unreacted monomers.

The DSC graph does not show any melting endothermic curve, wherefore the polymer is amorphous. The polymer Tg determined by DSC is -58.0°C. The thermogravimetric analysis (TGA) shows a weight loss of 10% at 307°C.

### EXAMPLE 16

### Terpolymer H-MOVE 2/H-MOVE 2a/TFE

In a reactor similar to that described in Example 10, 100 µl of perfluoropropionylperoxide at 6% by weight in CFCl₂-CF₂Cl and 5 mmoles of a H-MOVE 2 (88%) and H-MOVE 2a (12%) mixture and 18 mmoles of tetrafluoroethylene, are introduced.

The same procedure described in Example 10 is followed.

At the end of the degassing, the reactor is thermostated at the temperature of 30°C under magnetic stirring. The internal pressure decreases from 6.8 atm to 6.5 atm in about 6 hours (reaction time).

After distillation of the unreacted monomers, and polymer stripping under vacuum at 150°C for 3 hours, 300 mg of the polymer are separated.

By ¹⁹F-NMR analysis of the polymer dissolved under heating in C₆F₆ it is calculated that the molar percentage of the perfluorovinylethers (H-MOVE 2 + H-MOVE 2a) contained in the polymer is 33%. The H-MOVE 2/H-MOVE 2a molar ratio in the polymer is equal to the H-MOVE 2/H-MOVE 2a molar ratio of the fed mixture. Unreacted monomers are not evident.

The IR analysis does not show, in the polymer spectrum, absorption bands in the zone of the fluorinated double bonds. The DSC graph does not show any melting endothermic curve wherefore the polymer is amorphous. The Tg determined by DSC is -44.5°C.

The TGA shows a weight loss of 10% at 450°C.

### EXAMPLE 1 (comparative)

### Copolymer PVE/TFE

In a polymerization reactor identical to that described in Example 10, 250 µl of perfluoropropionylperoxide at 3% by weight in CFCl₂CF₂Cl, 9.8 mmoles of PVE and 18 mmoles of tetrafluoroethylene, are in sequence introduced.

The procedure already described in the previous Example 10 is followed until thermostating at the temperature of 30°C under magnetic stirring. The reaction time is of eight hours.

After distillation of the unreacted monomers and stripping under vacuum at 150°C for 3 hours, 540 mg of the polymer are recoverd.

By the ¹⁹F-NMR analysis carried out on the polymer dissolved in C₆F₆ it is calculated that the PVE molar percentage in the polymer is 23%.

The IR analysis shows that, in the polymer spectrum, there are absorption bands in the carboxyl zone, whose intensity is 10 times higher than that obtained from a MOVE 1/TFE copolymer film prepared according to Example 10, and having the same thickness.

The DSC graph does not show any melting endothermic curve, wherefore the polymer is amorphous. The TGA shows a weight loss of 2% at 427°C and of 10% at 463°C. The Tg, determined by DSC, is +15°C.

The polymer intrinsic viscosity, measured at 30°C in Fluorinert^{®} FC-75, is 51 ml/g.

### EXAMPLE 2 (comparative)

### Copolymer between β-PDE (CF₃₀CF₂CF₂OCF=CF₂)/TFE

In a polymerization reactor identical to that described in Example 10, 250 µl of perfluoropropionylperoxide at 3% by weight in CFCl₂-CF₂Cl, 10 mmoles of β-PDE and 18 mmoles of tetrafluoroethylene, are in sequence introduced.

The procedure described in the previous Example 10 is followed until the thermostating step at the temperature of 30°C under magnetic stirring.

By the ¹⁹F-NMR analysis carried out on the polymer purified from the monomers by the processes described in the previous Examples, it is calculated that the molar percentage of β-PDE in the polymer is 23%.

The DSC graph does not show any melting endothermic curve wherefore the polymer is amorphous. The Tg, determined by DSC, is -4.8°C.

This Tg value is clearly higher than those obtainable with the vinylethers of the invention (see the above Examples).

### EXAMPLE 3 (comparative)

### Crystalline copolymer PVE/TFE (PFA)

One operates as in Example 9 except that in this case the perfluoropropylvinylether (PVE) is used instead of α-PDE to obtain a copolymer having MFI equal to that of the Example 9.

The IR analysis shows absorption bands in the carboxyl zone, whose intensity is the double of that obtained from a TFE/MOVE 1 copolymer film having an equal thickness prepared according to Example 9.

## Claims

1. Fluorovinylethers having the following formula (I):
CFX=CXOCF₂OR (I)
wherein:
R is a C₂-C₆ linear, branched (per)fluorooxyalkyl group containing from one to three oxygen atoms; when R is a fluorooxyalkyl group as above defined it can contain from 1 to 2 atoms, equal to or different from each other, selected from the following: H, Cl, Br, I;
X = F.

2. Fluorovinylethers according to claim 1, having the following formula (II):
CFX=CXOCF₂OCF₂CF₂Y (II)
wherein:
Y = OCF₃;
X = F.

3. A synthesis process of the fluorovinylethers of claims 1 and 2, said process comprising:
the initial reaction of hypofluorite with a fluorinated olefin having the formula R₁R₂C=CR₃R₄ to give the intermediate hypofluorite F-CR₁R₂-CR₃R₄-OCF₂OF the subsequent reaction of this compound with a second fluorinated olefin of formula R₅R₆C=CR₇R₈ to give the intermediate F-CR₁R₂-CR₃R₄-OCF₂O-CR₅R₆-CR₇R₈-F, which by subsequent dehalogenation or dehydrohalogenation leads to the obtainment of the fluorovinylethers of claim 1 or 2, according to the scheme:
a)
CF₂(OF)₂ + R₁R₂C=CR₃R₄ → F-CR₁R₂-CR₃R₄-OCF₂OF (VI)
b)
F-CR₁R₂-CR₃R₄-OCF₂OF + R₅R₆C²=C¹R₇R₈→ F-CR₁R₂-CR₃R₄-OCF₂O-C²R₅R₆-C¹ R₇R₈-F (VII) 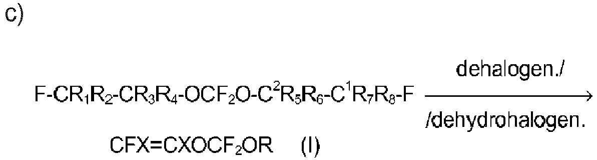
wherein:
- in the compound (VII):
- R₁ and R₄ are F; R₂, R₃, equal or different are H, Cl under the following conditions:
(1) when the final reaction is a dehalogenation, R₂, R₃ = Cl,
(2) when the final reaction is a dehydrohalogenation, one of the two substituents R₂, R₃ is H and the other is Cl;
- R₅, R₆, R₇, R₈ are: F or a C₁-C₄ linear or branched perfluorooxyalkyl group containing from one to three oxygen atoms;
- when one of the R₅ to R₈ radicals is a C₂-C₄ linear or branched fluorooxyalkyl group containing from one to three oxygen atoms, one or two of the other R₅ to R₈ are F and one or two of the remainders, equal to or different from each other, are selected from H, Cl, Br, Iodine; when the substituents selected from H, Cl, Br, Iodine are two, they are both linked to the same carbon atom;
- the fluorinated olefin used in the reaction a) is replaceable with that of the subsequent reaction b); in this case the meanings defined for the substituents of the R₁-R₄ group, and respectively of the R₅-R₈ group, are interchangeable each other, with the proviso that the position of each radical of each of the two groups R₁-R₄ and R₅-R₈ with respect to -OCF₂O-on the chain of the intermediate compound (VII), is the same which is occupied if the synthesis takes place according to the above reported scheme and the two olefins each react in the considered steps.

4. The process according to claim 3, wherein in step a) the reactants are in an approximately unitary molar ratio, or with an excess of CF₂(OF)₂, the residence time of the mixture in the reactor ranges from few hundredths of second up to about 120 seconds, the reaction temperature from -40 to -150°C and the compound (VI) is not separated and is transferred in a continuous way into the reaction of step b).

5. A process according to claims 3 and 4, wherein in the step b) the R₅R₆C=CR₇R₈ olefin in a pure state or in solution, reacts with the product obtained in the first reaction with formation of the compound (VII); the olefin is fed in a continuous way, so as to maintain its concentration constant in the reactor; the temperature of the reaction b) can range from -20° to -130°C, the olefin concentration is higher than or equal to 0.01 M.

6. The process according to claim 5, wherein the temperature of the reaction b) can range from -50°C to -100°C.

7. The process according to claim 5 or 6, wherein the olefin concentration is higher than 3 M.

8. The process according to claim 5 or 6, wherein the pure olefin is used.

9. The process according to claims 3 and 4, wherein in the step c) the compound (VII), dependently on the olefins used in steps a) and b), after distillation from the reaction raw material, is subjected to dechlorination or to dehydrochlorination to obtain the fluorovinylethers of formula (I).

## Patentansprüche

1. Fluorvinylether mit der folgenden Formel (I) :
CFX=CXOCF₂OR (I)
worin :
R für eine lineare oder verzweigte C₂-C₆-(Per)fluoroxyalkylgruppe mit einem bis drei Sauerstoffatomen steht; wenn R für Fluoroxyalkyl gemäß obiger Definition steht, kann es 1 bis 2 Atome, die gleich oder verschieden sind und aus H, Cl, Br und I ausgewählt sind, enthalten;
X=F.

2. Fluorvinylether nach Anspruch 1 mit der folgenden Formel (II) :
CFX=CXOCF₂OCF₂CF₂Y (II)
worin :
Y = OCF₃;
X=F.

3. Verfahren zur Synthese der Fluorvinylether gemäß den Ansprüchen 1-2, bei dem man :
zunächst Hypofluorit mit einem fluorierten Olefin mit der Formel R₁R₂C=CR₃R₄ zum Hypofluorit-Zwischenprodukt F-CR₁R₂-CR₃R₄-OCF₂OF umsetzt und diese Verbindung danach mit einem zweiten fluorierten Olefin der Formel R₅R₆C=CR₇R₈ zum Zwischenprodukt F-CR₁R₂-CR₃R₄-OCF₂O-CR₅R₆-CR₇R₈-F umsetzt, welches durch nachfolgende Dehalogenierung oder Dehydrohalogenierung zum Erhalt der neuen Fluorvinylether führt gemäß Anspruch 1 oder 2, gemäß dem Schema :
a)
CF₂(OF)₂ + R₁R₂C=CR₃R₄ → F-CR₁R₂CR₃R₄-OCF₂OF (VI)
b)
F-CR₁R₂-CR₃R₄-OCF₂OF + R₅R₆C²=C¹R₇R₈ → F-CR₁R₂-CR₃R₄-OCF₂O-C²R₅R₆-C¹R₇R₈-F (VII) 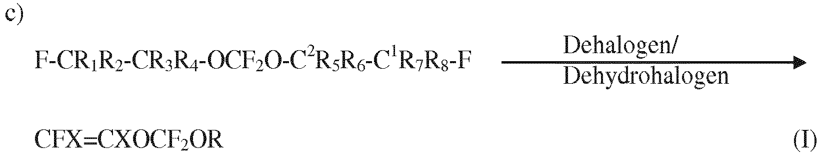
worin :
- in der Verbindung (VII) :
- R₁ und R₄ für F stehen; R₂ und R₃ gleich oder verschieden sind und für H oder Cl stehen, unter den folgenden Bedingungen :
(1) wenn es sich bei der letzten Reaktion um eine Dehalogenierung handelt, R₂, R₃ = Cl,
(2) wenn es sich bei der letzten Reaktion um eine Dehydrohalogenierung handelt, steht einer der beiden Substituenten R₂ und R₃ für H und der andere für Cl;
- R₅, R₆, R₇ und R₈ für F oder eine lineare oder verzweigte C₁-C₄-Perfluoroxyalkylgruppe mit einem bis drei Sauerstoffatomen stehen;
- wenn einer der Reste R₅ bis R₈ für eine lineare oder verzweigte C₂-C₄-Fluoroxyalkylgruppe mit einem bis drei Sauerstoffatomen steht, einer oder zwei der anderen Reste R₅ bis R₈ für F stehen und einer oder zwei der übrigen Reste gleich oder voneinander verschieden sind und aus H, Cl, Br und Iod ausgewählt sind; wenn zwei aus H, Cl, Br und Iod ausgewählte Substituenten vorhanden sind, diese beide an das gleiche Kohlenstoffatom gebunden sind;
- das bei der Reaktion a) verwendete fluorierte Olefin durch dasjenige der nachfolgenden Reaktion b) ersetzt werden kann; in diesem Fall sind die für die Substituenten der Gruppe R₁-R₄ definierten Bedeutungen und respektive der Gruppe R₅-R₈ untereinander austauschbar, mit der Maßgabe, dass die Position jedes Rests jeder der beiden Gruppen R₁-R₄ und R₅-R₈ bezüglich -OCF₂O- in der Kette der Zwischenverbindung (VII) die gleiche ist, die besetzt wird, wenn die Synthese gemäß dem oben angegebenen Schema erfolgt und die beiden Olefine jeweils in den betrachteten Schritten reagieren.

4. Verfahren nach Anspruch 3, bei dem in Schritt a) die Reaktanten in ungefähr unitärem Molverhältnis oder mit einem Überschuss an CF₂(OF)₂ vorliegen, die Verweilzeit der Mischung im Reaktor im Bereich von einigen Hundertstel einer Sekunde bis etwa 120 Sekunden beträgt, die Reaktionstemperatur -40 bis -150°C beträgt und die Verbindung (VI) nicht abgetrennt und kontinuierlich in die Reaktion von Schritt b) überführt wird.

5. Verfahren nach den Ansprüchen 3 und 4, bei dem in Schritt b) das Olefin R₅R₆C=CR₇R₈ in reinem Zustand oder in Lösung mit dem bei der ersten Reaktion erhaltenen Produkt unter Bildung der Verbindung (VII) reagiert; das Olefin kontinuierlich zugeführt wird, um seine Konzentration im Reaktor konstant zu halten; die Temperatur der Reaktion b) im Bereich von -20 bis - 130°C liegen kann, die Olefinkonzentration größer gleich 0,01 M ist.

6. Verfahren nach Anspruch 5, bei dem die Temperatur der Reaktion b) im Bereich von -50 bis -100°C liegen kann.

7. Verfahren nach Anspruch 5 oder 6, bei dem die Olefinkonzentration höher als 3 M ist.

8. Verfahren nach Anspruch 5 oder 6, bei dem das reine Olefin verwendet wird.

9. Verfahren nach den Ansprüchen 3 und 4, bei dem man in Schritt c) die Verbindung (VII) je nach den in den Schritten a) und b) verwendeten Olefinen nach Destillation aus dem Reaktionsrohmaterial einer Dechlorierung oder Dehydrochlorieuung unterwirft, wobei man die Fluorvinylether der Formel (I) erhält.

## Revendications

1. Éthers fluorovinyliques ayant la formule (I) suivante :
CFX=CXOCF₂OR (I)
dans laquelle :
R est un groupe (per)fluorooxyalkyle en C₂-C₆ linéaire ou ramifié contenant un à trois atomes d'oxygène ; quand R est un groupe fluorooxyalkyle tel que défini ci-dessus, il peut contenir 1 à 2 atomes, identiques ou différents l'un de l'autre, choisis parmi les suivants : H, Cl, Br, I ;
X=F.

2. Éthers fluorovinyliques selon la revendication 1, ayant la formule (II) suivante :
CFX=CXOCF₂OCF₂CF₂Y (II)
dans laquelle :
Y = OCF₃ ;
X=F.

3. Procédé de synthèse des éthers fluorovinyliques des revendications 1 et 2, ledit procédé comprenant :
la réaction initiale d'hypofluorite avec une oléfine fluorée ayant la formule R₁R₂C=CR₃R₄ pour obtenir l'hypofluorite intermédiaire F-CR₁R₂-CR₃R₄-OCF₂OF, la réaction consécutive de ce composé avec une deuxième oléfine fluorée de formule R₅R₆C=CR₇R₈ pour obtenir l'intermédiaire F-CR₁R₂-CR₃R₄-OCF₂O-CR₅R₆-CR₇R₈-F, qui par déshalogénation ou déshydrohalogénation consécutive conduit à l'obtention des éthers fluorovinyliques de la revendication 1 ou 2, selon le schéma :
a)
CF₂(OF)₂ + R₁R₂C=CR₃R₄ → F-CR₁R₂-CR₃R₄-OCF₂OF (VI)
b)
F-CR₁R₂-CR₃R₄-OCF₂OF + R₅R₆C²=C¹R₇R₈ → F-CR₁R₂-CR₃R₄-OCF₂OC²R₅R₆-C¹R₇R₈-F (VII) 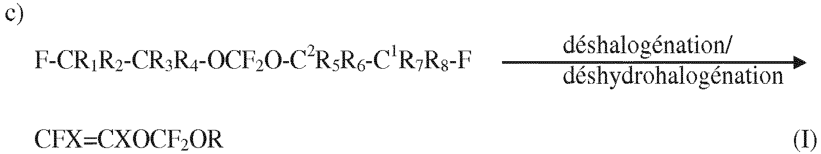
dans lequel :
- dans le composé (VII) :
- R₁ et R₄ représentent F ; R₂, R₃, identiques ou différents, représentent H, Cl dans les conditions suivantes :
(1) quand la réaction finale est une déshalogénation, R₂, R₃ = Cl,
(2) quand la réaction finale est une déshydrohalogénation, un des deux substituants R₂, R₃ est H et l'autre est Cl ;
- R₅, R₆, R₇, R₈ représentent : F ou un groupe perfluorooxyalkyle en C₁-C₄ linéaire ou ramifié contenant un à trois atomes d'oxygène ;
- quand un des radicaux R₅ à R₈ est un groupe fluorooxyalkyle en C₂-C₄ linéaire ou ramifié contenant un à trois atomes d'oxygène, un ou deux des autres R₅ à R₈ représentent F et un ou deux des restants, identiques ou différents l'un de l'autre, sont choisis parmi H, Cl, Br, l'iode ; quand les substituants choisis parmi H, Cl, Br, l'iode sont au nombre de deux, ils sont tous deux liés au même atome de carbone ;
- l'oléfine fluorée utilisée dans la réaction a) est remplaçable par celle de la réaction consécutive b) ; dans ce cas, les significations définies pour les substituants du groupe R₁-R₄, et respectivement du groupe R₅-R₈, sont interchangeables les unes avec les autres, à condition que la position de chaque radical de chacun des deux groupes R₁-R₄ et R₅-R₈ par rapport à -OCF₂O- sur la chaîne du composé intermédiaire (VII) soit la même que celle qui est occupée si la synthèse a lieu selon le schéma indiqué ci-dessus et les deux oléfines réagissent chacune dans les étapes considérées.

4. Procédé selon la revendication 3, dans lequel à l'étape a) les réactifs sont dans un rapport molaire approximativement unitaire, ou avec un excès de CF₂(OF)₂, le temps de résidence du mélange dans le réacteur va de quelques centièmes de seconde à environ 120 secondes, la température de réaction de -40 à -150 °C et le composé (VI) n'est pas séparé et est transféré de façon continue dans la réaction de l'étape b).

5. Procédé selon les revendications 3 et 4, dans lequel à l'étape b) l'oléfine R₅R₆C=CR₇R₈, dans un état pur ou en solution, réagit avec le produit obtenu dans la première réaction avec formation du composé (VII) ; l'oléfine est introduite de façon continue, de manière à maintenir sa concentration constante dans le réacteur ; la température de la réaction b) peut aller de -20° à -130 °C, la concentration d'oléfines est supérieure ou égale à 0,01 M.

6. Procédé selon la revendication 5, dans lequel la température de la réaction b) peut aller de -50 °C à -100 °C.

7. Procédé selon la revendication 5 ou 6, dans lequel la concentration d'oléfine est supérieure à 3 M.

8. Procédé selon la revendication 5 ou 6, dans lequel l'oléfine pure est utilisée.

9. Procédé selon les revendications 3 à 4, dans lequel à l'étape c) le composé (VII), en fonction des oléfines utilisées dans les étapes a) et b), après distillation à partir de la matière réactionnelle brute, est soumis à une déchloration ou à une déshydrochloration pour obtenir les éthers fluorovinyliques de formule (I).
